# EUROPÄISCHE PATENTSCHRIFT

(11) **EP 1 959 834 B1**
(45) Veröffentlichungstag und Bekanntmachung des Hinweises auf die Patenterteilung: **10.11.2010**
(21) Anmeldenummer: 06829137.6
(22) Anmeldetag: 27.11.2006
(51) Int. Cl.: A61B 5/151

(54) **GEKNICKTE LANZETTE**
KINKED LANCET
LANCETTE COUDEE

(30) Priorität: 25.11.2005 EP 05025739
(43) Veröffentlichungstag der Anmeldung: 27.08.2008
(73) Patentinhaber: Roche Diagnostics GmbH, 68305 Mannheim (DE); F. Hoffmann-La Roche AG, 4070 Basel (CH)
(72) Erfinder: HOENES, Joachim, 64673 Zwingenberg (DE); DECK, Frank, 67150 Niederkirchen (DE); HAAR, Hans-Peter, 69168 Wiesloch (DE); KRAEMER, Uwe, 68549 Ilvesheim (DE); ZIMMER, Volker, 67229 Laumersheim (DE); HARTTIG, Herbert, 67434 Neustadt (DE)
(86) Internationale Anmeldenummer: PCT/EP2006/011341
(87) Internationale Veröffentlichungsnummer: WO 2007/060004

(56) Entgegenhaltungen:
- EP-A- 1 402 812
- EP-A- 1 508 304
- US-A- 2 801 633
- US-A- 5 395 388
- US-A1- 2004 193 202
- US-A1- 2005 245 845

## Beschreibung

### Technisches Gebiet

Die Erfindung liegt auf dem Gebiet der Stechhilfen zur diagnostischen Ermittelung von Blutparametern.

### Stand der Technik

Die Gewinnung und Analyse von Körperflüssigkeiten findet auf vielen Gebieten der medizinischen Diagnostik statt. Deshalb ist es wünschenswert auch Routinetests außerhalb des Laboratoriums schnell und reproduzierbar zu ermöglichen. Das Testen kann mit verschiedenen Körperflüssigkeiten durchgeführt werden, wie z. B. Blut und/oder interstitielle Flüssigkeit. Diese Flüssigkeiten können auf verschiedene Charakteristiken hin untersucht werden. Die Ergebnisse dieser Untersuchung sind wichtig, um verlässliche Diagnosen, therapeutische Maßnahmen und Therapieverfolgungen durchführen zu können.

Die Analyse von Körperflüssigkeiten beginnt mit der Gewinnung der Flüssigkeit. Eine Methode zur Gewinnung von Körperflüssigkeit besteht darin, eine minimale Wunde in die Haut des Patienten mit Hilfe einer Nadel, Lanzette oder eines Messers zu erzeugen. Die dabei gewonnene Körperflüssigkeit kann entweder in kleinen Gefäßen gesammelt werden oder direkt in Kontakt mit einem Testelement wie z. B. einem Teststreifen zur Analyse gebracht werden. Um bei der Benutzung der Lanzetten, Nadel oder Klingen eine Verletzungsgefahr des Patienten zu vermeiden, wird die Stechhilfe mit einem Schutz an der Lanzettenspitze konstruiert. Die meisten dieser Stechhilfen benötigen ein manuelles Einfügen der Lanzette in die Stechhilfe. Dies ist bei einer sehr häufigen Benutzung der Stechhilfe eine sehr umständliche Handhabung. Eine Magazinierung von Lanzetten könnte dieses Problem beheben, wobei hier viele Sicherheitsaspekte zu beachten sind. Es ist beispielsweise zu beachten, dass die Sicherheit des Patienten bei der Benutzung der Stechhilfe gewährleistet ist. Zudem sollte das System nicht zu komplex werden, da es sonst vom Patienten nicht gut handhabbar wäre.

Im Stand der Technik werden hierzu einige Lösungen aufgezeigt. Das US Patent 2003/0199902 gewährleistet eine Versiegelung jeder einzelnen Lanzette in einem Magazin, wobei ein aufwendiger und Raum erfüllender Zahnradmechanismus dazu benutzt wird, die Lanzetten aus dem Magazin zu befördern.

In der europäischen Anmeldung EP 1 203 563 wird ein analytisches Hilfsmittel beschrieben, das auf einem Trägerband ein Testelement aufweist, wobei auf diesem Testelement ein zusätzliches Rahmenelement aufgebracht ist, das beweglich ist und eine Lanzette beinhaltet. Das Rahmenelement kann bei Benutzung von einer parallelen Stellung zum Testelement in eine orthogonale Stellung bewegt werden, so dass die Lanzette durch eine Öffnung im Testelement aktuiert werden kann. Dies ist eine recht aufwendige Realisierung von einer Kombination von Testelement und Lanzette, da viele Teile mechanisch bewegt werden müssen und das System in seiner funktionsfähigen Form viel Platzbedarf hat.

Eine weitere europäische Anmeldung mit der Nummer EP 1 360 935, beschreibt eine Anordnung von Lanzetten (hier als "Tester" bezeichnet), um Zugang zu flüssigen Proben zu erhalten. Die Lanzetten sind dabei seriell auf einem Band angeordnet, das auf seiner Oberseite eine Abdeckung der Lanzetten aufweist. Um die Lanzette zur Benutzung freizulegen, wird auch hier ein aufwendiges mechanisches System benutzt, da der gesamte Lanzettenkörper zunächst aus der Bandebene heraus bewegt werden muss, um die Lanzette benutzen zu können.

Dieser Stand der Technik weist diverse Nachteile auf. Es sind viele mechanische Schritte notwendig, um das einzelne Stechelement aus der Magazinierung, bei der die Lanzetten in einer seriellen Anordnung, also in der Trägerbandebene liegen, in eine Anordnung zu bewegen, bei der die Lanzette senkrecht zur Trägerbandebene angeordnet ist. Dies hat aufgrund der aufwendigen Mechanik zusätzlich den Nachteil, dass ein großer Platzbedarf für diese Mechanik benötigt wird. Ein weiterer Nachteil vieler Systeme aus dem Stand der Technik ist die aufwendige Entsiegelung der Lanzetten vor dem Stechvorgang.

Aus den Nachteilen des Standes der Technik ergibt sich folgende Aufgabe. Es soll eine Platz sparende, mit wenig mechanischem Aufwand benutzbare, magazinierbare Stechhilfe zur Verfügung gestellt werden, die eine einfache Handhabung möglich macht.

Diese Aufgabe wird durch den Gegenstand der Erfindung, wie er in den unabhängigen Patentansprüchen charakterisiert wird, gelöst. Bevorzugte Ausführungsformen sind Gegenstand der abhängigen Ansprüche.

Gegenstand der Erfindung ist eine Vorrichtung zur Gewinnung von Körperflüssigkeit, die mindestens eine Lanzette beinhaltet, wobei die Lanzette mindestens aus einem Lanzettenkörper und einer Lanzettenspitze (im Folgenden auch Spitze genannt) besteht. Erfindungsgemäß weist die Lanzette einen Knickbereich auf, sodass die Lanzette unter Krafteinwirkung in dem Bereich der Spitze geknickt wird, sodass die Spitze in Bezug auf die Längsachse des Lanzettenkörpers in ihrer Ausrichtung verändert wird.

Das Anknicken der Lanzettenspitze aus der Lanzettenkörperebene wird auch als erste Knickbewegung bezeichnet.

Die Lanzette besitzt eine längliche Ausdehnung, wobei ein Ende, hier das distale Ende genannt, zum Zwecke des Einstechens in einen Körper besonders ausgeformt ist, beispielsweise in Form einer Spitze. Die Spitze ist dabei ein Punkt der sich am distalen Ende der Lanzette befindet, in den die Seitenflächen des länglichen Lanzettenkörpers zusammenlaufen. Die in der Spitze mündenden Seitenflächen der Lanzette können dabei zusätzlich geschärfte Kanten aufweisen. Die Lanzette besteht also mindestens aus einem Lanzettenkörper, der vorwiegend nahezu parallel verlaufende Seitenflächen- bzw. kanten aufweist und einem Bereich der Spitze oder Spitzenbereich, der sich direkt an den Lanzettenkörper anschließt und aufeinander zu laufende Seitenkanten aufweist, die in der Spitze münden. Der Bereich der Spitze, oder auch Spitzenbereich, kann also je nach Länge der auf einander zu laufenden Seitenkanten, unterschiedlich groß sein.

Zum Abknicken der Lanzettenspitze weist die Lanzette einen Knickbereich auf. Dieser Knickbereich dient dazu mindestens eine Stelle in der Lanzette vorzuhalten, der geeignet ist, unter möglichst geringer Krafteinwirkung ein Abknicken mindestens der Lanzettenspitze zu ermöglichen. Dieser Knickbereich kann sich über Teile des Lanzettenspitzenbereiches als auch über Teile des Lanzettenkörpers erstrecken. Der Knickbereich weist mindestens eine Struktur mit geänderter Steifigkeit auf, die es ermöglicht, die Lanzette an dieser Stelle leicht zu Verformen. Bevorzugter Weise beginnt der Knickbereich im Spitzenbereich und erstreckt sich über einen Teil des Lanzettenkörpers, der vor allem in seiner Breite eine Verlängerung des Spitzenbereiches darstellt, jedoch im Wesentlichen mindestens zwei parallel verlaufende Kanten aufweist. Der weitere Teil des Lanzettenkörpers, der sich an den Knickbereich anschließt und im proximalen oder hintere Ende der Lanzette mündet, kann eine von dem Knickbereich verschiedene Geometrie aufweisen, wie beispielsweise eine Verbreiterung oder Verdickung des Lanzettenkörpers. Dieser breitere Teil des Lanzettenkörpers kann zusätzlich härter gearbeitet sein, um eine höhere Stabilität gegen Verformung aufzuweisen. Dies kann beispielsweise durch die Wahl anderer Materialien geschehen oder durch die Wahl der Menge bzw. Dicke der verwendeten Materialien. Ist die Lanzette auf einem Träger angebracht, so ist mindestens der hintere Lanzettenkörperteil, der vorzugsweise eine festere Struktur gegen Verformung aufweist mit dem Träger verbunden, um eine stabile Verbindung mit dem Träger herstellen zu können. Der hintere Lanzettenkörperteil kann bevorzugter Weise zur Ankopplung einer Antriebseinheit benutzt werden. Hierfür kann der Lanzettenkörper unterschiedliche Ankopplungsstrukturen aufweisen, wie beispielsweise Nuten, Kerben oder Ausstülpungen. Dabei findet der Antrieb vorzugsweise quer zur Ausrichtung des Lanzettenkörpers statt, sodass die abgeknickte Lanzettenspitze linear in ein Körperteil eingestochen werden kann. Durch die Ausrichtung der abgeknickten Lanzettenspitze, die von der Längsachse des Lanzettenkörpers verschieden ist ergibt sich der Vorteil, dass andere geometrische Anordnungen realisiert werden können als das mit der rein axialen Antriebsrichtung für Lanzettenkörper und Lanzettenspitze möglich ist. Des Weiteren ergibt sich durch das bevorzugte Abknicken der Lanzettenspitze, die aufgrund ihrer Zweckbestimmung (möglichst schmerzarm in ein Körperteil eingestochen zu werden), möglichst dünn und fein ausgestaltet ist, dass nur wenig Kraft hierzu notwendig ist bzw. wenig morphologische Veränderung an der Lanzette vorgenommen werden muss, um ein leichtes Abknicken zu ermöglichen. Hierdurch wird sowohl eine einfache Abknickung der Lanzette gewährleistet, ohne dass eine erhöhte Labilität der Lanzette bei einer möglichen Magazinierung auf einem Träger oder Trägerband eintritt. Der Lanzettenkörper, der zum Ankoppeln der Antriebseinheit dient, kann unabhängig der Ausgestaltung des abknickbaren Spitzenbereiches stabil ausgefertigt sein, um den Beanspruchungen bei der Ankopplung der Antriebseinheit und den Kräften bei der Stechbewegung zu genügen. Ein Abknicken der Lanzette außerhalb des Knickbereiches könnte zu einer Destabilisierung der Lanzettenstruktur führen.

Mindestens die Lanzettenspitze kann mit einem Sterilschutz versehen sein, der bevorzugter Weise beim Abknicken der Lanzettenspitze geöffnet wird.

In einer Ausführungsform befindet sich die Lanzette auf einem Träger. Der Träger kann beispielsweise zur einfachen Magazinierung von mehreren Lanzetten dienen. Außerdem kann der Träger auch eine Schutzfunktion gegenüber äußeren Einflüssen (wie beispielsweise Stößen oder sonstige Berührung) für die Lanzette übernehmen, wenn die Lanzette beispielsweise zu mindestens einem Teil vom Träger umgeben ist. Dies ist besonders bevorzugt, wenn der Träger eine Trägerband ist. In einer bevorzugten Ausführungsform sind der Lanzettenkörper und die Lanzettenspitze im ungeknickten Zustand auf dem Träger, vorzugsweise liegend, angebracht. Besonders bevorzugt liegt mindestens der Bereich der Spitze vollständig auf dem Träger auf.

Als Träger kann alternativ eine kreisförmige Struktur dienen, an oder auf der die mindestens eine Lanzette befestigt ist. Bevorzugter Weise ist der Träger scheibenförmig ausgestaltet. Es sind aber auch andere Trägerstrukturen, die beispielsweise eckige, kugel- oder bandförmige, ovale oder elliptische Formen aufweisen, denkbar.

Die Lanzette und der Träger können in einem Stück ausgebildet sein. Dies ist bevorzugt, wenn die gesamte Struktur aus Metall, besonders bevorzugt aus Stahl gefertigt wird. Aber auch andere Materialien, wie beispielsweise Keramik oder Polymerstrukturen wären für eine einstückige Ausbildung von Lanzette und Träger möglich.

In einer alternativen Ausführungsform beinhaltet die Lanzette eine Struktur, die zur Aufnahme von Körperflüssigkeit geeignet ist. Dies kann bevorzugter weise eine Kapillarstruktur sein, aber auch alle alternativen Strukturen, wie beispielsweise Lochstrukturen, Spalt- oder Rinnenstrukturen sind zur Aufnahme von Flüssigkeit geeignet. Dabei kann auch durch eine Prägung im Knickbereich eine gebildete Struktur dazu ausgebildet sein, Flüssigkeit aufzunehmen. Diese Ausführungsform wird im Folgenden als Microsampler bezeichnet, da eine Probenaufnahme durch die Lanzette und nicht direkt durch ein Testelement erfolgt. Die Struktur zur Aufnahme von Körperflüssigkeit kann sich bevorzugter Weise im Spitzenbereich befinden. Sie kann in einer alternativen Ausführungsform auch über den Spitzenbereich hinausgehen und sich über Teile des Lanzettenkörpers erstrecken. Dabei kann die Struktur zur Aufnahme der Körperflüssigkeit in einem Stück ausgebildet sein oder in mehrere Bereiche unterteilt sein. In einer bevorzugten Ausführungsform beginnt diese Struktur zur Aufnahme von Flüssigkeit im Spitzenbereich und erstreckt sich über annähernd die gleiche Ausdehnung wie im Spitzenbereich in den Lanzettenkörper hinein. Hierbei kann die Struktur zur Aufnahme von Körperflüssigkeit in den Knickbereich münden oder über den Knickbereich hinaus in den Lanzettenkörper hineinragen. Die im Microsampler gesammelte Körperflüssigkeit kann anschließend auf ein Testelement übertragen werden und durch ein Detektionssystem (z. B. optisch oder elektrochemisch) detektiert und durch ein Auswertesystem ausgewertet werden.

Des Weiteren kann mindestens ein Testelement in, auf oder neben diesem Träger oder einem weiteren Träger angeordnet sein. Das Testelement dient zur Aufnahme der gewonnen Körperflüssigkeit und der anschließenden Detektion eines Analyten aus der Körperflüssigkeit. Dabei kann das Testelement Reagenzien zur Reaktion mit dem Analyten enthalten. Das Testelement kann auf einem separaten Träger angebracht sein, oder auf dem Träger der Lanzette. Um eine Kontamination der Spitze mit Substanzen aus dem Testelement zu vermeiden ist das Testelement bevorzugter Weise nicht direkt mit der Lanzette verbunden sondern separat von der Lanzette auf dem Träger angeordnet. Die Anordnung des mindestens einen Testelements auf einem separaten Träger verstärkt diesen Effekt der verringerten Kontaminationsgefahr. In einer bevorzugten Ausführungsform sind Testelement und Lanzette so zueinander angeordnet, dass sie nach dem Knicken der Lanzettenspitze durch eine zweite Bewegung der Lanzette oder des Testelementes in Kontakt treten können. Dies ist besonders bevorzugt, wenn die Lanzette in Form eines Microsamplers ausgebildet ist. Dies kann durch eine Bewegung der Lanzette relativ zum Träger durchgeführt werden. Eine Möglichkeit die Lanzette mit dem Testelement in Kontakt zu bringen ist das weitere Abknicken der Lanzette in die erste Knickrichtung, sodass die Lanzettenspitze mehr als 90° in Bezug auf den Lanzettenkörper abgeknickt ist. Das Testelement befindet sich in dieser Ausführungsform bevorzugter Weise auf einem Teil des Lanzettenkörpers. Eine alternative Bewegung der Lanzette zum Kontaktieren der Lanzette mit einem Testelemente, ist eine Auslenkbewegung der Lanzette oder des Trägers in die, zur ersten Knickbewegung, entgegen gesetzten Richtung. Bei dieser Bewegung kann die Lanzettenspitze in die Ebene des Lanzettenkörpers zurückgeknickt werden. Alternativ zur Kontaktierung von Testelement und Lanzette kann die Körperflüssigkeit auch direkt vom Körperteil des Benutzers auf das Testelement übertragen werden.

Eine weitere Alternative das Testelement mit der Lanzette zu kontaktieren ist die Bewegung des Testelementes selber. Hierzu befindet sich das Testelement bevorzugter weise auf einem zweiten Träger, wobei zumindest zum Teil der Träger der Lanzette oder des Testelementes relativ zueinander beweglich angeordnet sind.

In einer bevorzugten Ausführungsform ist der Träger als Trägerband ausgestaltet, auf dem bevorzugt eine Mehrzahl Lanzetten positioniert sind. In dieser Ausführungsform wird eine Vorrichtung zur Gewinnung von Körperflüssigkeiten beschrieben, die ein im Wesentlichen planares Trägerband mit einer Längsausrichtung und einer Querausrichtung besitzt, auf der mindestens eine Lanzette, beinhaltend einen Lanzettenkörper und eine Spitze angeordnet ist, wobei die Lanzette liegend auf dem Trägerband, angeordnet ist. Wie bereits beschrieben ist die Vorrichtung **dadurch gekennzeichnet, dass** die Lanzette eine Struktur mit veränderter Steifigkeit (gegenüber dem restlichen Lanzettenmaterial) beinhaltet, die wie bereits erwähnt als Knickbereich bezeichnet wird.

Die Steifigkeit soll als Maß für den Widerstand des Materials gegen elastische Verformung verstanden werden. Bevorzugter weise besitzt diese Struktur erniedrigte Steifigkeit gegenüber dem restlichen Lanzettenkörper, so dass die Lanzette unter Krafteinwirkung bevorzugt in diesem Bereich geknickt wird. Dabei wird die Spitze in Bezug auf den restlichen Lanzettenkörper in ihrer Ausrichtung verändert. Diese Änderung der Ausrichtung ist bevorzugter weise aus der Trägerbandebene bzw. Lanzettenkörperebene heraus. Dabei bleibt der Lanzettenkörper mindestens zu einem Teil in der ürsprünglichen Ebene bzw. der Trägerbandebene und ist daran fixiert. Die Kraft, die zur Veränderung der Ausrichtung der Lanzettenspitze benötigt wird, wird auch als Schwellenkraft bezeichnet. Diese Schwellenkraft soll so groß sein, dass sie die Veränderung der Ausrichtung der Lanzettenspitze bewirkt, ist dabei aber so dimensioniert, dass keine ungewollten Verformungen an Lanzette, am Träger oder am Trägerband auftreten.

Die Übertragung der Kraft auf die Lanzette kann durch ein Knickelement, z.B. einem Stößel stattfinden, der auf die Lanzette gedrückt wird. Bei einer speziellen Ausführungsform mit mehr als einer Prägung, besteht die Möglichkeit die Kraft auf die Lanzette zu übertragen indem der Träger oder das Trägerband mit der Lanzette über den Stößel geleitet wird. Hier wirkt eine ausreichend große Kraft (Schwellenkraft) auf die Lanzette, um die Lanzettenspitze aus der Lanzettenkörperebene bzw. aus der Trägerbandebene herauszubewegen. Bei Lanzetten auf oder an einem Träger oder Trägerband, verbleibt zumindest ein Rest des Lanzettenkörpers am Trägerband verbleibt.

In einer bevorzugten Ausführungsform ist das Knickelement zweiteilig. Hierbei wird die Lanzettenspitze durch den Stößel aus der Trägerbandebene bzw. der Lanzettenkörperebene herausbefördert, indem der Lanzettenkörper an sich oder zusammen mit dem Trägerband durch den zweiten Teil des Knickelements an einer Bewegung in Richtung der Stößelbewegung gehindert wird. Dieser zweite Teil des Knickelements kann beispielsweise eine Sperre sein, die sich bevorzugter weise auf der dem Stößel gegenüberliegenden Seite des Trägers, Trägerbandes oder der Lanzette befindet. Sowohl der Stößel als auch die Sperre können zusätzlich durch ein Regelelement gesteuert werden, sodass die Position des Knickes variiert werden kann. Auf diese Weise kann die Lanzette an verschiedenen Stellen geknickt werden und es entstehen unterschiedlich lange Spitzen zum Einstechen in die Haut. Die Kraftübertragung vom Stößel auf die Lanzette ist besonders leicht möglich, wenn der Spitzenbereich der Lanzette mit dem Träger oder Trägerband nicht fest verbunden ist. Besonders bevorzugt für die Anordnung auf einem Träger oder Trägerband ist dabei eine Flachlanzette.

Der Knickbereich der Lanzette, der auch außerhalb des Spitzenbereiches liegen kann, weist mindestens eine Struktur mit veränderter Steifigkeit auf. Diese mindestens eine Struktur mit veränderter Steifigkeit wird wie bereits erwähnt als Prägung bezeichnet. Die Prägung kann durch z. B. Stanzen oder Hämmern oder sonstige Metall bearbeitende Maßnahmen in oder auf die Lanzette gearbeitet werden. Die Steifigkeit kann also vorzugsweise durch die Variation der Geometrie eines Bauteils oder durch die Variation der Materialmenge im Bauteil eingestellt werden. Eine bevorzugte Ausführungsform beinhaltet mehr als eine Prägung in dem Knickbereich der Lanzette. Eine besonders bevorzugte Ausführungsform dieser Prägung ist eine Dreifachprägung im Knickbereich der Lanzette, die sich über mindestens einen Teil der Längsausdehnung der Lanzette erstreckt. Eine Prägung erstreckt sich dabei von dem distalen Ende der Lanzette in axialer Richtung in Richtung zum proximalen Ende der Lanzette. Die Länge der Prägung ist variabel. Diese Prägung kann von zwei Seiten in die Flachlanzette eingebracht sein. Diese unterschiedliche Richtung der Prägung bewirkt, dass die Lanzettenspitze in die entgegen gesetzte Richtung zum Lanzettenkörper abknickt.

Der erste Teil der ersten Prägung befindet sich im Spitzenbereich. Dieser erste Teil der Prägung kann auf den Spitzenbereich beschränkt sein, aber auch darüber hinausgehen. Eine zweite Prägung grenzt an das proximale Ende des ersten Teils der ersten Prägung in Richtung Seitenkante der Lanzette an. Eine dritte Prägung verläuft ebenfalls angrenzend an das proximale Ende des ersten Teils der ersten Prägung in Richtung der gegenüberliegenden Seitenkante der ersten Prägung der Lanzette. Die zweite und dritte Prägung werden von der gleichen Seite eingeprägt wie der erste Teil der ersten Prägung. Durch die Ausrichtung dieser Prägungen ist es möglich, mittels einer niedrigen Schwellenkraft auf die Lanzette, ein Abknicken der an die mindestens eine Prägung angrenzenden Flächen, zu bewirken. Hierdurch werden die abgeknickten Flächen in einem Winkel von vorzugsweise bis zu 100 ° aus der Lanzettenkörper- bzw. Trägerbandebene gehoben. Dadurch wird die Lanzettenspitze aus der Ebene des Lanzettenkörpers oder des Bandes herausbewegt.

Das Material der Lanzette ist bevorzugter weise Metall, besonders bevorzugt ist Stahl. Die Lanzette kann aber auch aus anderen Materialien bestehen, die es sowohl ermöglichen, dass die Lanzette bei Krafteinwirkung knickbar ist und dabei genügend Steifigkeit besitzt, um bei Benutzung in die Haut einzudringen ohne ihre Form zu verändern. Außerdem sollte das Material der Gestalt sein, dass es an dem distalen Ende der Lanzette zu einer scharfen Spitze gearbeitet werden kann, da sonst beim Stich zu viel Schmerz generiert wird. Die Herstellung von Lanzetten im Allgemeinen ist im Stand der Technik hinreichend bekannt, wie beispielsweise in DE19604156 oder EP0565970.

Das Trägerband ist bevorzugter weise aus einer Plastikfolie gefertigt. Dies kann aber auch ein anderes flexibleres Material sein, wie z.B. in der Anmeldung US 20050245845 beschrieben. In einem integrierten System kann auf dem Trägerband zusätzlich mindestens ein Testelement angeordnet sein. Bevorzugt werden Lanzette und Testelement alternierend angeordnet. Die Lanzette kann sowohl diagonal, in Längsausrichtung wie auch in Querausrichtung auf dem Band angebracht sein. Eine mögliche Ausführungsform ist die Anordnung von Lanzette und Testelement in direkter Nachbarschaft. Auf diese Weise ist ein direkter Transfer von Flüssigkeit nach dem Stechvorgang auf das Testelement möglich, ohne dass das Band weiter bewegt werden muss.

Zur Aktuierung der Lanzette werden im Folgenden verschiedene Möglichkeiten beschrieben. Die Lanzette kann an ihrem proximalen Ende auf dem Träger oder Trägerband so fixiert sein, dass ein Teil der Lanzette in Relation zum oder mit dem Träger oder Trägerband bewegt werden kann, während das proximale Ende an mindestens einem Punkt mit dem Träger oder Trägerband verbunden ist. Eine weitere bevorzugte Befestigung der Lanzette, ist die Fixierung des Lanzettenkörpers an dem Träger oder Trägerband, wobei sich der Spitzenbereich vom Träger oder Trägerband löst. Die gesteuerte Bewegung der Lanzette kann durch Bewegen des Träger oder Trägerbandes oder durch Ergreifen der Lanzette mit einem Greifelement geschehen, wobei die Lanzettenspitze mit dem Träger oder Trägerband aus der Ebene des Träger oder Trägerbandes herausbewegt wird. Diese Bewegung kann mittels eines Antriebselementes durchgeführt werden, das senkrecht zur Träger oder Trägerbandebene auf die Lanzette Kraft überträgt. Die Kraftübertragung findet durch ein Antriebselement statt, das z.B. ein Stößel sein kann oder ein Greifelement, welches die Lanzette an ihrem Lanzettenkörper greift und bewegt. Hierbei ist in einer bevorzugten Ausführungsform die Einstichtiefe des Blutentnahmegerätes frei wählbar. Zur Regulierung der Einstichtiefe wird die Bewegung der Lanzette durch ein variierbares Anschlagselement definiert, gegen das die Lanzette während des Einstichvorgangs anschlägt. In Abhängigkeit von der Position des Anschlagelementes wird auf diese Weise die Länge der Lanzettenspitze, die aus der Gehäuseöffnung austritt und somit die Einstichtiefe variiert. Das Anschlagselement kann beispielsweise in das Gehäuse integriert werden. Des Weiteren kann die Lanzette selbst als Anschlagselement dienen, wobei die Stechtiefe durch die Länge der abgeknickten Spitze definiert wird. Da die Lanzette in abgeknicktem Zustand in einem von 0° abweichenden Winkel zum Lanzettenkörper abgeknickt ist, kann der Lanzettenkörper eine Barriere für das weitere Eindringen der Lanzette in die Haut darstellen. So ist es möglich, Lanzetten mit mehreren Prägungen im Knickbereich zu benutzen, um durch die Wahl der Prägung, die zum Knicken benutzt wird, die Stechtiefe zu verändern.

Zum Antrieb der Lanzette können ballistische oder Kulissen geführte Mechanismen benutzt werden, die aus dem Stand der Technik bekannt sind, wie z.B. in DE19604156, EP0565970, US5318584 oder US4924879 beschrieben. Eine bevorzugte Ausführungsform für den Antrieb der Lanzette ist die freie Bewegung der Lanzette nach Kraftübertragung durch das Antriebselement, wie beispielsweise dem Stößel. In dieser Ausführungsform wird ein Impuls von dem Antriebselement auf die Lanzette übertragen und die Lanzette bewegt sich ohne weitere Führung durch das Antriebselement in Richtung Gehäuseöffnung. Die Bewegung der Lanzette kann dabei durch zusätzliche Elemente am Gehäuse geführt werden.

Zur hygienischen Verwendung des Systems wird die Lanzette mindestens im Spitzenbereich durch einen Sterilschutz geschützt. Bevorzugter weise ist die Lanzette über den ganzen Lanzettenkörper mit dieser Folie überdeckt. Die Folie kann sich dabei auch über einen Teil des Trägerbandes bzw. Trägers erstrecken und ist damit verbunden. Dieser Sterilschutz kann aus einer Polymerschicht bestehen, die nach der Verknüpfung der Lanzette mit dem Trägerband bzw. Träger aufgebracht wird. Der Sterilschutz wird beim Aufwenden der Schwellenkraft auf die Lanzettenspitze von der Lanzettenspitze zerstört oder durchstochen und legt einen Teil der Lanzette, aber mindestens den Spitzenbereich der Lanzette frei. Alternativ kann der Sterilschutz vor Benutzung der Lanzette entfernt werden. Bevorzugter weise wird der Sterilschutz hierbei im Ganzen entfernt.

Gegenstand der Erfindung ist ebenfalls ein System zur Gewinnung von Körperflüssigkeit. Dieses System besteht bevorzugter weise aus einem Gehäuse, in dem ein Träger bzw. ein im Wesentlichen planares Trägerband montiert ist, und mindestens einer Lanzette, die vorzugsweise liegend auf dem Träger oder Trägerband angeordnet ist. Das Gehäuse weist mindestens eine Öffnung auf, durch die die Lanzette bei Aktuation hindurch treten kann. Das im Wesentlichen planare Trägerband ist bevorzugter weise auf zwei Spulen aufgewickelt. Es können aber auch andere Bevorratungsmöglichkeiten zur Magazinierung der benutzten und unbenutzten Lanzetten eingesetzt werden, wie bereits in Form eines Trägers beschrieben. Bei Verwendung von zwei Spulen zur Magazinierung der Lanzetten befinden sich die unbenutzten Lanzetten auf der einen Spule und die benutzten Lanzetten auf der anderen Spule. Die Lanzetten bestehen aus einem Material, das weich genug ist, um auf dem Trägerband aufgewickelt zu werden, ohne dabei geknickt zu werden. Andererseits ist das Material der Lanzetten so stabil, dass die Lanzette bei Aktuation und Eintritt in die Haut nicht verformt wird. Alternativ werden die Lanzetten quer auf dem Trägerband angeordnet, so dass ein Biegen der Lanzette vermieden werden kann. Eine weitere Möglichkeit ein Biegen der unbenutzten Lanzetten zu vermeiden, ist die Wahl des Durchmessers der Spule auf dem die Lanzetten aufbewahrt werden, sodass die Lanzetten beim Aufrollen kaum gebogen werden.

Die Lanzette weist eine Lanzettenspitze auf, die sich am distalen Ende der Lanzette befindet. Im System befindet sich ein Knickelement, das auf die Lanzette so einwirkt, dass die Lanzettenspitze in Bezug auf den restlichen Lanzettenkörper in ihrer Ausrichtung veränderbar ist. In einer bevorzugten Ausführungsform kann das Knickelement bei Krafteinwirkung auf die Lanzette vor der Aktuation, den Ort der Krafteinwirkung auf den Lanzettenkörper regeln. Hierzu kann das Knickelement durch ein Regelelement gesteuert werden. Zur Kraftübertragung kann ein Stößel dienen.

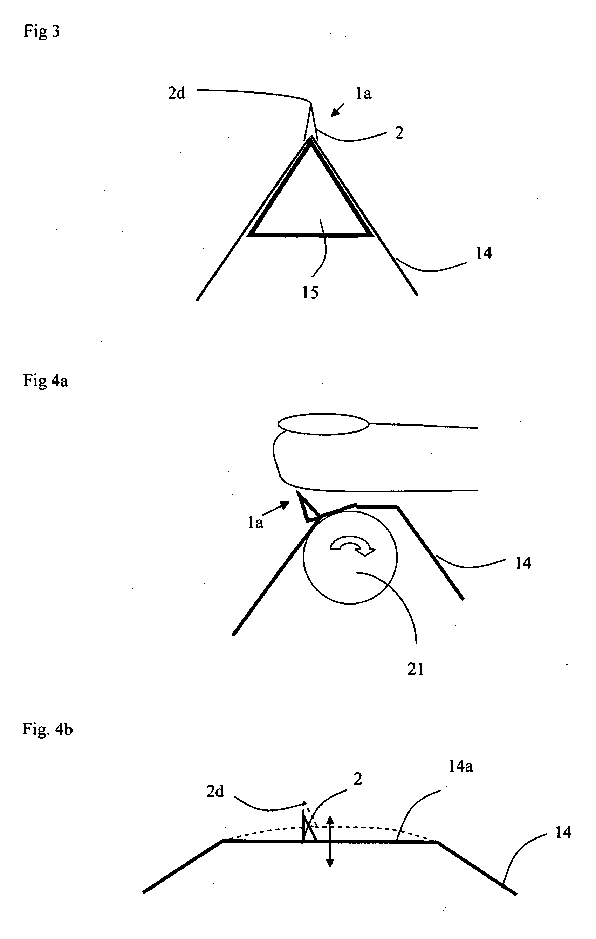

Eine weitere Ausführungsform des Knickelementes ist ein Stößel, über den die Lanzette, der Träger oder das Trägerband mit der Lanzette geleitet wird, sodass die Kraft, die dabei auf die Lanzette wirkt ausreicht, um die Lanzettenspitze umzuknicken. Um eine gute Kraftübertragung auf die Lanzette zu erreichen, kann beispielsweise das Trägerband gespannt werden. Weitere Ausführungsformen für ein einstückiges Knickelement für das Trägerband ist beispielsweise ein Rad mit einem möglichst kleinen Radius (s. Figur 4a), bzw. eine Führung über eine Kante (s. Figur 3), über die das Trägerband geleitet wird. Die Funktionsweise dieser Alternativen wird bei der Beschreibung der Figuren näher ausgeführt.

In dem System bzw. der Vorrichtung können herkömmliche Lanzetten benutzt werden, bevorzugter weise Flachlanzetten, aber auch alle Lanzetten, bei denen die Schwellenkraft des Knickelementes ausreicht, um die Spitze der Lanzette aus der Ebene des Trägerbandes bzw. der Lanzettenkörperebene herauszubewegen. Die Lanzette wird nach oder während dieses Knickvorgangs von einem Antriebselement in Richtung Gehäuseöffnung bewegt, um anschließend den Stechvorgang auszuüben. Dabei tritt mindestens ein Teil der Lanzette aus der Gehäuseöffnung aus und sticht in die Haut des Patienten. An der Einstichstelle bildet sich ein Bluttropfen, der zur Analyse benutzt wird. Wenn sich ein Testelement auf dem Träger oder Trägerband befindet, wird der Träger oder das Trägerband, wenn nötig so weit transportiert, dass des Testelement sich unterhalb der Gehäuseöffnung befindet. Der Bluttropfen kann auf das Testelement aufgebracht werden, ohne dass der Patient weitere Schritte einleiten muss. Alternativ kann sich das Testelement auch auf einem zweiten Träger befinden, wie bereits beschrieben. Das Blut reagiert mit einem oder mehreren Reagenzien, die sich auf dem Testelement befinden, wie sie z. B. aus den Dokumenten EP-A 0 885 591, EP-B 0 535 480 und EP-B 0 477 322 bekannt sind. Das Testelement wird mittels eines Detektors analysiert.

Das Blut kann auf verschiedene Komponenten hin untersucht werden, wie es im Stand der Technik bekannt ist. Zum Beispiel kann die Analyse auf Blutbestandteile wie Hämatokrit, Glucose, Cholesterin, Koagulation, Eisen und andere gerichtet sein. Zur Analyse können unterschiedliche Methoden zur Anwendung kommen. So können beispielsweise elektrochemische Nachweisreaktionen benutzt werden, aber auch optische (z.B. Reflektion, Absorption, Fluoreszenz, Raman-Spektroskopie) oder magnetische Nachweisreaktionen. Typischerweise wird die Flüssigkeit mit einem Testsystem in Kontakt gebracht, wobei eine Reaktion zwischen einem Testelement und der Flüssigkeit stattfindet. So beruht die Detektion mittels eines optischen Testelements auf einer Farbreaktion zwischen Flüssigkeit und Nachweisreagenz. Beispiele für diese Reaktionen sind in den US Patenten 3,802,842; 4,061,468 und 4,490,465 beschrieben.

Bei der Benutzung des Gerätes führt das System verschiedene Schritte durch. Die Lanzette wird in eine Position gebracht, in der sie durch Einwirkung einer Schwellenkraft auf den Lanzettenkörper in den geknickten Zustand gebracht wird. Dabei wird vorzugsweise der Sterilschutz von der Lanzette durchbrochen. Die Lanzette wird, wenn nötig, bis zur Öffnung des Gehäuses transportiert. Dort wird sie mit Hilfe eines Antriebselements aktuiert und tritt dabei zu einem Teil aus der Gehäuseöffnung aus. Bei dem Aktuationsvorgang tritt die Lanzette zumindest zu einem Teil in die Haut des Patienten ein und danach wieder in das Gerät zurück. Dabei kann im Falle der Verwendung eines Microsamplers Blut an der Lanzette gesammelt werden. Wird ein Transportband verwendet, wird dieses weiter transportiert und auf eine Spule gewickelt. Dabei liegt die Lanzette bevorzugter Weise wieder flach auf dem Trägerband. Dieser Remagazinierungsvorgang wird in der Patentanmeldung US 20050245845 beschrieben.

In einem integrierten System, in dem auch Testelemente auf dem Träger oder Trägerband, vorzugsweise alternierend mit den Lanzetten aufgebracht sind, wird das Testelement nach dem Stechvorgang bis zur Gehäuseöffnung transportiert, um den Bluttropfen zur Analyse aufzunehmen. Das Testelement kann bis zum Detektor transportiert und dort vermessen werden. Im Falle der Verwendung eines Microsamplers wird das gesammelte Blut auf ein benachbartes Testelement übertragen. Wie bereits erwähnt, kann sich das Testelement auf dem gleichen Träger befinden oder auf einem zweiten Träger. Die beiden Träger sind dabei vorzugsweise beweglich zueinander angeordnet.

Weitere bevorzugte Ausführungsformen wären eine Vorrichtung, bei der sich mindestens eine Struktur mit geänderter Steifigkeit über einen Teil der Längsausdehnung des Spitzenbereiches erstreckt und die Lanzette an ihrem proximalen Ende auf dem Trägerband fixiert sein könnte, wobei der Träger und die Lanzette in einem Stück ausgeformt sein könnten. Lanzettenkörper und Lanzettenspitze könnten beispielsweise im ungeknickten Zustand, vorzugsweise liegend, auf dem Träger angebracht sein. Der Knickbereich könnte im Spitzenbereich und einem Teil des Lanzettenkörpers liegen, wobei der hintere Teil des Lanzettenkörpers zum Ankoppeln einer Antriebseinheit dienen könnte und die Antriebsrichtung der Lanzette im Wesentlichen parallel zur abgeknickten Lanzettenspitze und quer zur Lanzettenkörperebene liegen würde. Weiterhin würden das Testelement und die Lanzette im ungeknickten Zustand nicht in direktem Kontakt miteinander stehen, wobei Lanzette und Testelement nebeneinander auf oder an dem Träger angeordnet sein könnten. Weiterhin würde mindestens die Lanzettenspitze einen Sterilschutz besitzen, wobei dieser vor oder während der Benutzung der Lanzette von der Lanzettenspitze getrennt werden würde. Des Weiteren würde ein Sterilschutz mindestens den Spitzenbereich der Lanzette überdecken oder umgeben, und der Sterilschutz würde beim Knicken der Lanzette durch die Lanzettenspitze zerstört oder durchstochen werden. Insbesondere der Knickbereich würde mehrere Bereiche mit geränderter Steifigkeit aufweisen, wobei beim Knicken durch Wahl des Bereiches mit geränderter Steifigkeit die Länge der abgeknickten Spitze variierbar wäre und die abgeknickte Lanzettenspitze würde im Wesentlichen wieder in die Ebene des Lanzettenkörpers zurückgeknickt werden. Beispielsweise könnte die Lanzettenspitze durch ein in die erste Knickrichtung gerichtetes Weiterknicken in die Lanzettenkörperebene geknickt werden.

### Kurzbeschreibung der Figuren

Figur 1a: Schematische Darstellung einer Lanzette mit einer Prägung in unbenutztem Zustand.
Figur 1b: Schematische Darstellung einer Lanzette mit einer Prägung in geknicktem Zustand.
Figur 1c: Schematische Darstellung einer Flachlanzette mit mehreren Prägungen in unbenutztem Zustand.
Figur 1d: Schematische Darstellung einer Lanzette mit mehreren Prägungen in geknicktem Zustand.
Figur 2a: Schematische Darstellung der Queranordnung von Lanzetten auf dem Trägerband.
Figur 2b: Schematische Darstellung der Lanzetten in Längsanordnung auf dem Trägerband.
Figur 3: Schematische Darstellung von Lanzette und Band und deren Führung im Längsschnitt.
Figur 4a: Schematische Darstellung der Aktuation der Lanzette in dem das Trägerband über eine Rolle geführt wird.
Figur 4b: Schematische Darstellung der Lanzette in Aktuation wobei das Band horizontal aus seiner Ebene bewegt wird.
Figur 5a: Schematische Darstellung des Trägerbandes mit alternierender Anordnung von Testfeldern und längs angeordneten Lanzetten.
Figur 5b: Schematische Darstellungen des Trägerbandes mit alternierender Anordnung von Testfeldern und quer angeordneten Lanzetten.
Figur 6: Schematische Darstellung eines integrierten Gerätes mit Gehäuse und allen wichtigen Komponenten.
Figur 7: Schematische Darstellung eines Multilanzettenrades in der Aufsicht
Figur 8 a-d: Lanzette des Multilanzettenrades aus Figur 7 in ungeknicktem Zustand und 3 verschiedenen Kickgraden

### Beschreibung der Figuren

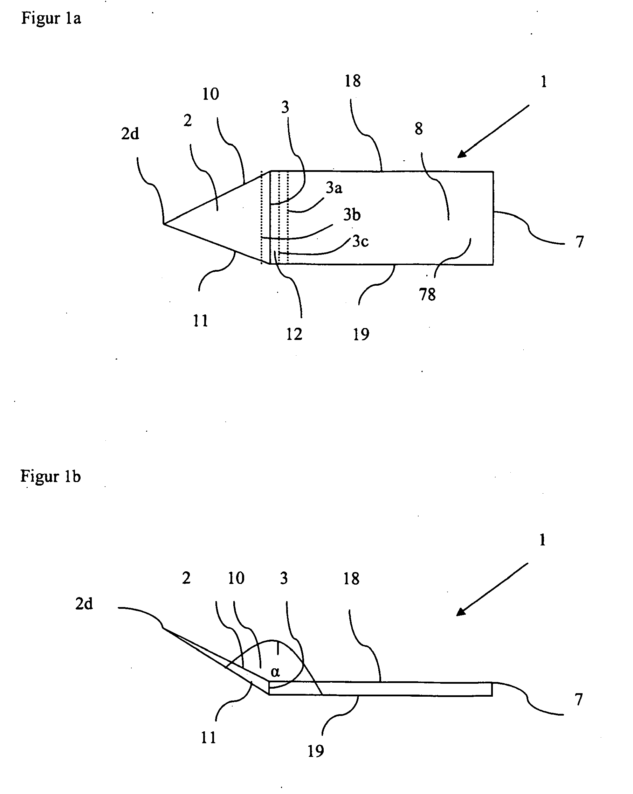

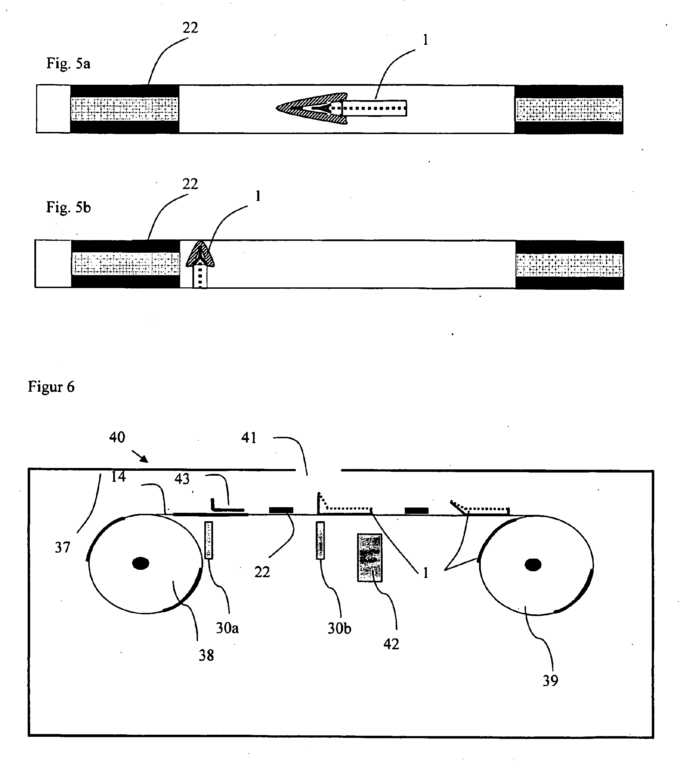

Figur 1a stellt eine mögliche Ausführungsform der Lanzette (1) dar. Die Lanzette (1) besitzt ein distales Ende (2) und ein proximales Ende (7). Die Lanzette (1) besitzt einen Bereich (8), der sich an das proximale Ende (7) anschließt und in den Spitzenbereich (2) übergeht. Die Lanzette (1) weist eine Struktur (3) mit einer geänderten Steifigkeit auf, die im Folgenden als Prägung bezeichnet wird. Die Prägung (3) befindet sich im Knickbereich, der innerhalb oder außerhalb des Spitzenbereiches (2) liegen kann. Diese Prägung (3) kann sich an verschiedenen Stellen (3a), (3b), (3c) zwischen distalem und proximalem Ende der Lanzette befinden. Der Knickbereich (12) wird durch den Bereich der Prägungen (3, 3a, 3b, 3c) definiert und kann je nach Anzahl und Lage der Prägungen variieren. Mindestens eine Prägung (3, 3a, 3b, 3c) liegt dabei innerhalb des Spitzenbereiches (2). Der Knickbereich kann sich weiterhin über einen Teil des Lanzettenkörpers (78) erstrecken. Der Knickbereich liegt dabei angrenzend an den Spitzenbereich (2). Der Spitzenbereich (2) mündet in der Lanzettenspitze (2d). Beim Abknicken der Lanzette, bewirkt ein Knickelement das durch ein Regelelement gesteuert wird (hier nicht gezeigt, s. Fig. 6), dass die Lanzette an einer der Prägungen (3) bzw. (3a), (3b), (3c) abgeknickt wird. Je nach Lage und Wahl des Knicks kann die Lanzette (1) beim Stechvorgang unterschiedlich tief in die Haut eindringen.

Eine Lanzette (1) mit einer Prägung (3) wird in Figur 1b im geknickten Zustand gezeigt. Die Lanzettenspitze(2d) sowie der Spitzenbereich (2) sind um den Winkel α zum Bereich (8) abgewinkelt. Der Winkel α kann zwischen 180° und ca. 80° liegen. Ein bevorzugter Bereich liegt zwischen 150° und 110°.

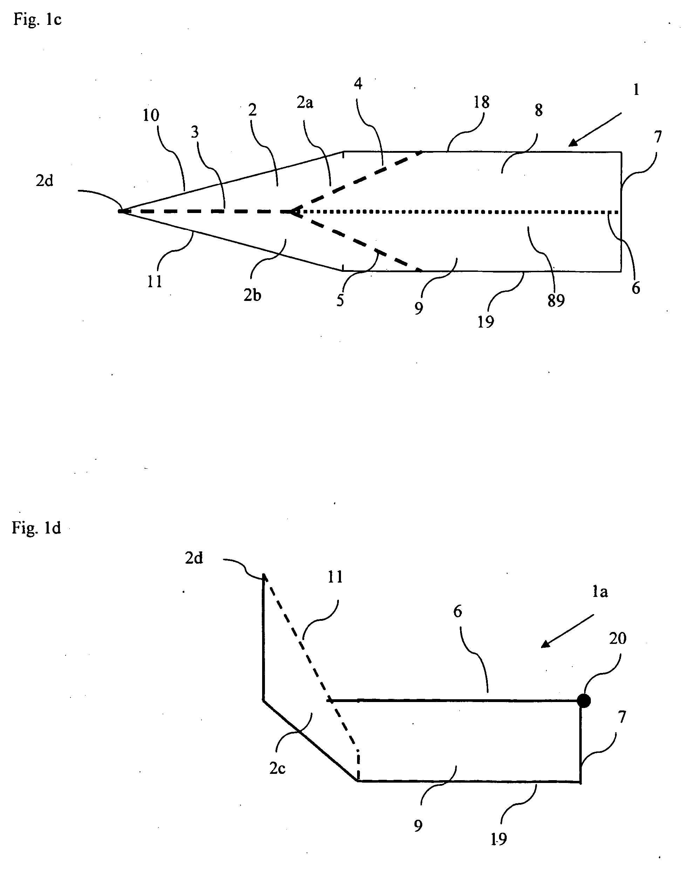

In Figur 1c wird eine Lanzette (1) dargestellt die eine bevorzugte Ausführungsform der Erfindung darstellt. Diese Lanzette (1) besitzt vorzugsweise mindestens eine Struktur mit einer geänderten Steifigkeit. Diese geänderte Steifigkeit dient zur leichteren und gezielten Veränderung der Ausrichtung der Lanzettenspitze in Bezug auf den restlichen Lanzettenkörper. Bevorzugter weise ist die Steifigkeit in dieser Struktur erniedrigt, sodass bei Einwirkung einer Kraft auf den Lanzettenkörper, den Spitzenbereich (2) mit der Lanzettenspitze (2d) an der Stelle der erniedrigten Steifigkeit abknickt. Eine solche Struktur veränderter Steifigkeit kann durch verschiedene Methoden erreicht werden. So kann beim Herstellungsprozess der Lanzette (1) an dieser Stelle weniger Material eingebracht werden. Eine weitere Möglichkeit wäre ein Stanz- oder Hammerprozess zum Einbringen der Struktur oder eine Prägevorgang. Es sind weiterhin alle im Stand der Technik bekannten Metall verarbeitenden Methoden verwendbar, die zu einer Struktur mit veränderter Steifigkeit führen. In einer bevorzugten Struktur ist die erste Prägung (3) vom distalen Ende (2d) der Lanzette (1) zumindest über einen Teil des Spitzenbereiches (2) in Richtung des proximalen Endes (7) der Lanzette (1) in das Material eingeprägt. Diese Prägung (3) kann sich bis an das proximale Ende (7) der Lanzette erstrecken. Lateral von dieser Prägung aus kann eine zweite Prägung (4) in das Material eingebracht sein. Diese Prägung kann innerhalb oder außerhalb des Spitzenbereiches (2) beginnen, die sich von der Mitte (6) der Lanzette (1) in Richtung Seitenrand (18) erstreckt. Der Winkel zwischen dieser Prägung (4) und der Mittelinie (6) der Lanzette (1) α beträgt zwischen 0° und 90°. Bevorzugter Weise liegt dieser Winkel (α) zwischen 30° und 70°. Eine dritte Prägung (5) erstreckt sich auf der gegenüber liegenden Seite zu der Prägung (4). Auch diese Prägung (5) erstreckt sich von der Mitte (6) der Lanzette (1) hin zum Seitenrand (19). Der Winkel zwischen der Mittellinie (6) und der Prägung (5) liegt ebenfalls zwischen 0° und 90°, wobei ein bevorzugter Bereich zwischen 30° und 70° liegt. Die Winkel α und β müssen nicht identisch sein. Der Übergang der Seitenkanten (10) und (11) in die Seitenkanten (18) und (19) der Lanzette bilden die Grenze des Spitzenbereiches. Dabei treffen sich die beiden Seitenkanten (10) und (11) in der Spitze (2d).Der Knickbereich kann innerhalb, aber auch außerhalb des Spitzenbereiches (2) der Lanzette liegen und erstreckt sich in einer bevorzugten Ausführungsform über die ganze Länge der Lanzette (1). Um die Prägungen in eine Lanzette (1) einbringen zu können, wird vorzugsweise eine Flachlanzette benutzt, die aus einem dünnen Blech besteht.

Durch die Prägungen (3), (4), (5) in das Blech entstehen Knicklinien. Diese Knicklinien ergeben zumindest teilweise Knickung des Bleches zur einen und teilweise zur anderen Seite der Lanzettenebene (89) aus Figur 1c. Die Lanzettenebene (89) wird aus den Flächen (8) und (9) der ungeknickten Lanzette (1) gebildet. In Figur 1c liegt die Lanzettenebene (89) in der Papierebene. Die geknickte Lanzette ist in Figur 1d in der Seitenansicht dargestellt, sodass die Lanzettenebene (89) um 90° aus der Papierebene gedreht ist. Das gezielte Knicken der Lanzette (1) kann auch durch Perforation oder Ritzung oder Ätzung entlang der Linien (3), (4), (5) erreicht werden. Durch die spezielle Anordnung der Knicklinien (3), (4), (5) ergibt sich bei Krafteinwirkung auf die Lanzette ein Abknicken der Spitze (2d) bis über 90° zur Lanzettenfläche (89) und gleichzeitig ein Abknicken der Seitenflächen (2a) und (2b) des Spitzenbereiches (2) sowie ein entgegen gesetztes Abknicken der Flächen (8) und (9) des restlichen Lanzettenkörpers. Diese bevorzugte Ausführungsform weist eine hohe Stabilität des Lanzettenkörpers auf, trotz der Struktur mit erniedrigter Stabilität. Die Lanzette (1) ist ausreichend stabil, um einen Stechvorgang zur Blutgewinnung in die Haut eines Patienten durchzuführen. In ungeknickten Zustand, vor oder nach einem Stich, kann die Lanzette bevorzugter weise auf ein Band aufgerollt werden, wie in der Patentanmeldung US 20050245845 beschrieben. Der Stich kann zum Beispiel durch Drehung der Lanzette um das Lanzettenende (20) ausgeführt werden. Die Prägungen (3), (4) schließen eine Fläche (2a) ein, während die Prägungen (3), (5) eine Fläche (2b) einschließen. In dem Ausführungsbeispiel, dargestellt in Figur 1c enden die Prägungen (4), (5) außerhalb des Spitzenbereiches (2) der durch die Seitenkanten (10), (11) abgegrenzt wird. Diese Linien verlaufen über den Spitzenbereich hinaus in den Kanten (18), (19) bis zum proximalen Ende (7) der Lanzette (1). In einer bevorzugten Ausführungsform könnten zusätzlich zu den Prägungen (4) und (5) weitere Prägungen vorzugsweise parallel zu den Prägungen (4) und (5) in den Knickbereich eingearbeitet sein (nicht in den Figuren gezeigt). Mit Hilfe dieser alternativen Prägungen kann die Spitze an unterschiedlichen Stellen abgeknickt werden und dadurch unterschiedliche Längen aufweisen. Auf diese Weise können unterschiedliche Stechtiefen in die Haut ermöglicht werden.

In Figur 1d ist die Lanzette in geknicktem Zustand dargestellt. In dieser Seitenansicht ist nun nur noch ein Teil der Flächen zu erkennen, bei dem Knickvorgang werden die Flächen (2a), (2b) nach oben aus der Lanzettenebene (89) gebogen während die Flächen (8), (9) sich nach unten aus der Ebene (89) bewegen. Die Mittellinie (6) bleibt dabei vorzugsweise in der Lanzettenebene (89). Die Knickung der Lanzettenspitze (2d) kann bis zu 100° zur Fläche der Lanzettenebene (89) stattfinden. Dabei wird in der seitlichen Ansicht die Unterseite des Spitzenbereiches (2) sichtbar, die in Figur 1d als Fläche (2c) zu erkennen ist. Diese bildet die Rückseite der Fläche (2b) in Figur 1c. In der Seitenansicht der Figur 1d nicht zu erkennen, sind die Flächen (8) und (2a). Die Fläche (8) liegt hinter der Fläche (9), während sich Fläche (2a) hinter der Fläche (2c) versteckt. Bei Drehung der Lanzette (1) im Uhrzeigersinn um den Drehpunkt (20), führt die Lanzette (1) eine Bewegung nach oben und bei Rückdrehung nach unten aus. Ein alternativer Antrieb der Lanzette (1) wäre durch Ergreifen des Lanzettenschaftes am proximalen Ende (7) der Lanzette (1) mit einem Greifer oder einer Zange möglich. Hierbei würde die Lanzette (1) nicht um einen Punkt (20) gedreht sondern im Ganzen bewegt. In dieser Ausführungsform sollte das Trägerband (14) eine ausreichende Flexibilität aufweisen, um die Bewegung der Lanzette nicht einzuschränken.

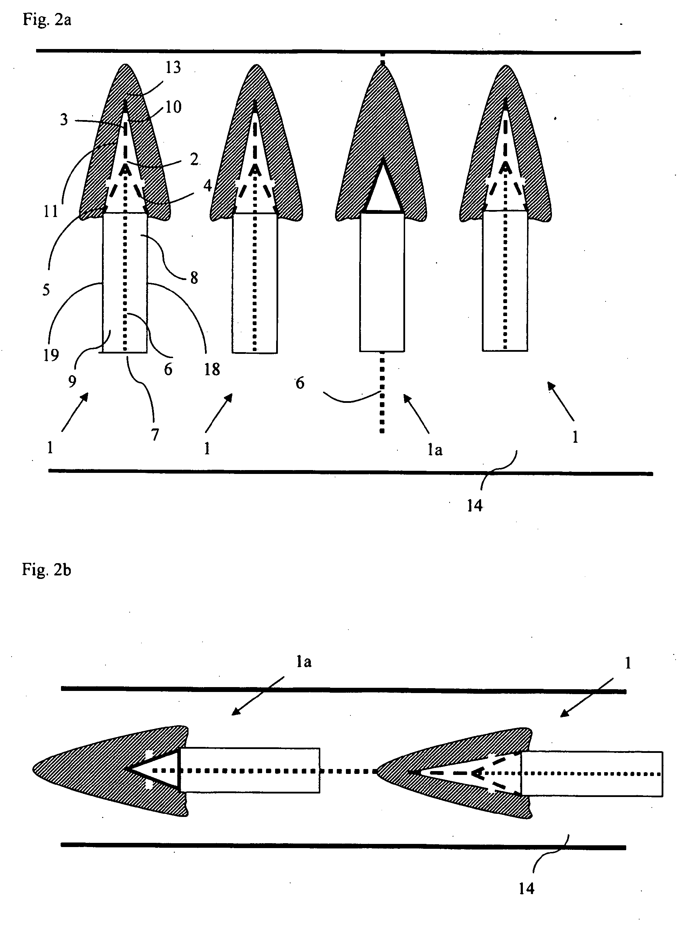

Figur 2a zeigt die Lanzetten (1) nach ihrer Herstellung. In dieser speziellen Ausführung sind die Lanzetten (1) durch Stanzen oder Ätzen aus einem dünnen Blechband, das hier das Trägerband (14) darstellt, herausgearbeitet. Die Lanzetten (1) sind auf dem Trägerband (14) quer zueinander angeordnet. Im Spitzenbereich (2) weisen sie die Prägungen (3), (4) und (5) auf, wobei die Prägungen (4) und (5) am proximalen Ende der Kanten (10) und (11) enden. Um den Spitzenbereich herum erstreckt sich ein Hohlraum (13), der durch Ausstanzen oder Ätzen gefertigt wird. Durch diesen Hohlraum (13) im Trägerband (14) um den Spitzenbereich (2) herum, kann die Spitze (2d) aus der Lanzettenebene herausgeknickt werden, wie dies für eine Lanzette (1a) dargestellt ist. Bei dieser Lanzette (1a) ist die Spitze (2d) nach oben geknickt, dabei stellt die Lanzettenmittellinie (6) die Biegelinie dar.

In Figur 2b sind die Lanzetten (1) auf dem Trägerband (14) in Längsausrichtung angeordnet. In Figur 2a ist sowohl eine ungeknickte Lanzette (1) als auch eine geknickte Lanzette (1a) dargestellt. Bei der geknickten Lanzette (1a) ist die Lanzettenspitze (2d) aus der Trägerbandebene (14) herausgeknickt.

Figur 3 ist eine schematische Darstellung der Anordnung von Lanzette (1) zu Trägerband (14) sowie zu einer Führung (15) des Trägerbandes (14). Die Führung (15) ist hier in einem gleichseitigen Dreieck dargestellt, wobei das Trägerband (14) über eine Kante (16) geleitet wird. Hierbei ist zu erkennen, dass die Lanzettenspitze (2d), in dem Moment, in dem die Lanzette (1) die Umlenkkante (16) erreicht, aus der Trägerbandebene herausgeknickt wird. Diese Möglichkeit Lanzetten zu knicken, ist besonders bevorzugt bei Lanzetten, wie sie in Figur 1c beschrieben werden, einzusetzen.

Figur 4a zeigt eine weitere Möglichkeit das Trägerband (14) so zu führen, dass die Lanzettenspitze (2d) automatisch aus der Trägerbandebene (14) herausgeknickt wird. In diesem Fall wird das Trägerband (14) über eine Rolle (21) geführt, die je nach Anordnung des Trägerbandes (14) auf der Rolle (21) entweder rechts oder links herum gedreht werden kann. In diesem Ausführungsbeispiel erstreckt sich die Lanzette (1) in Längsausrichtung auf dem Band (14) und das proximale Ende (7) der Lanzette (1) bewegt sich der Lanzettenspitze (2d) voraus. Die Rolle (21) kann beispielsweise aus einem profilierten Rad bestehen, das ein Rutschen des Trägerbandes (14) auf der Rolle (21) verhindert. Der Stechvorgang erfolgt hierbei durch schnelles Vor- und Zurückdrehen des Rades (21). Das Abknicken der Lanzette (1) kann hierbei durch ein zusätzliches Knickelement (z. B. eine Wulst, hier nicht dargestellt) auf dem Rad (21) erleichtert werden. Durch diese Wulst wird zusätzlich zur Kraft durch die Drehbewegung des Rades (21) eine Kraft auf die Mitte (6) des Lanzettenkörpers ausgeübt. Diese Kraft bewirkt das Knicken der Seitenflächen (8) und (9) des Lanzettenkörpers und damit das Abknicken der Lanzettenspitze (2d).

In Figur 4b ist eine geknickte Lanzette (1a) gezeigt, deren Spitze (2d) aus der Trägerbandebene (14a) herausragt. Um den Stechvorgang durchführen zu können, kann das Band (14) durch einen Bolzen (hier nicht dargestellt) auf der, der Lanzette (1a) gegenüber liegenden Seite, in Richtung Lanzettenspitze (2d) bewegt werden. Eine weitere Möglichkeit die Lanzette (1a) zu bewegen, ist mit Hilfe einer Zange (hier nicht dargestellt), die den Schaft der Lanzette (1a) ergreift und mittels einer auf und ab Bewegung den Stechvorgang durchführt. Eine bevorzugte Ausführungsform für diesen Stechvorgang ist es, dass Band (14) vor dem Stechvorgang zu spannen. Die Elastizität des Trägerbandes ist bevorzugter weise so zu wählen, dass es um die Stechtiefe (ca. 2 - 3 mm) ausgelenkt werden kann. Dabei kann mit Hilfe der Variation der Krafteinwirkung auf das Trägerband die Auslenkung des Trägerbandes verändert werden und somit die Stechtiefe (bzw. Austrittsweite der Lanzette) variiert werden.

In Figur 5a wird ein Trägerband (14) gezeigt, auf dem alternierend ein Testfeld (22) und eine Lanzette (1) angeordnet sind. Dabei ist die Lanzette (1) in Längsausrichtung zum Trägerband angebracht. Der Abstand zwischen dem Testfeld (22) und der Lanzette (1) auf dem Trägerband (14) kann variieren. So ist es möglich, die Lanzette (1) so dicht neben dem Testfeld (22) zu platzieren, dass nach dem erfolgten Einstich die Flüssigkeit sofort von dem Testfeld (22) aufgenommen werden kann, ohne das Trägerband (14) zu bewegen. Eine weitere Ausführungsform mit alternierenden Testfeldern (22) und Lanzetten (1) ist in Abbildung 5b gezeigt. Hierbei ist die Lanzette (1) quer auf dem Trägerband angeordnet. Auch hier kann die Lanzette (1) in variablem Abstand zum Testfeld (22) platziert werden.

In Figur 6 ist ein integriertes System gezeigt. Das System besteht aus einem Gerät (40), das bevorzugter weise ein Gehäuse (37) mit einer Öffnung (41) sowie ein Trägerband (14), auf dem die Lanzetten (1) befestigt sind, besitzt. Das Trägerband (14) ist auf 2 Spulen (38) und (39) aufgewickelt. Dabei befindet sich der unbenutzte Teil der auf dem Trägerband befestigten Lanzetten auf der Spule (38) und der benutzte Teil wird auf die Spule (39) gewickelt. Zwischen den Spulen (38, 39) ist das Trägerband (14) gestreckt. Die Spulen (38, 39) werden durch einen Antrieb bewegt wie er aus dem Stand der Technik bekannt ist. Bevorzugter weise wird nur eine der beiden Spulen (38, 39) angetrieben. Ein Beispiel für einen solchen Antrieb ist in der Anmeldung US 20050245845 beschrieben. Die Lanzetten (1) befinden sich im ungeknickten Zustand auf dem Trägerband (14), wenn das Trägerband (14) auf den Spulen gewickelt ist. Zwischen den beiden Spulen (38, 39) befindet sich ein erster Stößel (30a), der dazu dient die Kraft für den Knickvorgang auf die Lanzette (1) zu übertragen. Dieser Stößel (30a) ist unterhalb des Trägerbandes (14) angeordnet. Damit die Kraft nicht ausschließlich zur Auslenkung des Trägerbandes (14) dient, befindet sich oberhalb des Trägerbandes (14) gegenüber dem Stößel (30a) ein Knickelement (43), das das Trägerband (14) an einer vertikalen Bewegung hemmt. Das Knickelement (43) kann, je nach Anordnung der mindestens einen Prägung auf der Lanzette, unterschiedliche Formen aufweisen. Bei einer Lanzette mit nur einer Prägung ist keine besondere Form des Knickelements nötig, da das Knickelement in dieser Ausführungsform ausschließlich die Funktion hat, die Lanzette mit dem Trägerband an einer weiteren Bewegung zu hindern. In einer bevorzugten Ausführungsform des Knickelements (43) bei der eine Lanzette mit mehr als einer Prägung geknickt werden soll, wie sie in Figur 1c dargestellt ist, weist das Knickelement (43) eine Form auf, bei der die abzuknickenden Flächen (8) und (9) zwar an einer Bewegung gehindert werden, aber der Knickbereich mit der mindestens einen Prägung (6) nicht. Ein Beispiel für diese Anordnung ist ein Knickelement (43) mit zwei Flügeln, die sich über die abzuknickenden Flächen erstrecken, aber zwischen den Flügeln genug Raum bleibt, dass die Lanzette sich in diesen Raum weiter bewegen kann und geknickt wird.

Das Knickelement (43) kann ein Regelelement beinhalten (hier nicht gezeigt), das die Position des Knickelementes (43) so verändern kann, dass die Lanzette an unterschiedlichen Stellen geknickt wird. Dies ist besonders bevorzugt, bei Ausführungsformen, die nur eine Prägung (3) bzw. (3a, 3b, 3c) zum Abknicken der Lanzettenspitze benutzen.

Ein zweiter Stößel (30b), der sich unterhalb der Gehäuseöffnung (41) befindet, dient dazu die Lanzette bei der Aktuation anzutreiben. Zwischen Gehäuseöffnung (41) und Stößel (30b) befindet sich das Trägerband (14). Zum Auslösen des Stechvorgangs wird das Trägerband (14) soweit transportiert, bis eine unbenutzte Lanzette (1) zwischen Gehäuseöffnung (41) und Stößel (30b) liegt. Beim Auslösen des Stechvorgangs wird der Stößel (30b) mit so viel Kraft auf die Lanzette (1) zu bewegt, dass mindestens die Lanzettenspitze (2) aus der Gehäuseöffnung (41) heraus bewegt wird. Nach erfolgtem Einstich wird das Blut auf einem Testfeld (22) gesammelt. Dort findet eine Reaktion des Blutes mit den Reagenzien auf dem Testfeld statt, die mit Hilfe eines Detektors (42) analysiert werden kann. Die Lanzette (1) wird zusammen mit dem Trägerband (14) remagaziniert. Durch den Wicklungsvorgang auf der Spule (39) wird die Lanzette wieder flach in das Trägerband (14) integriert.

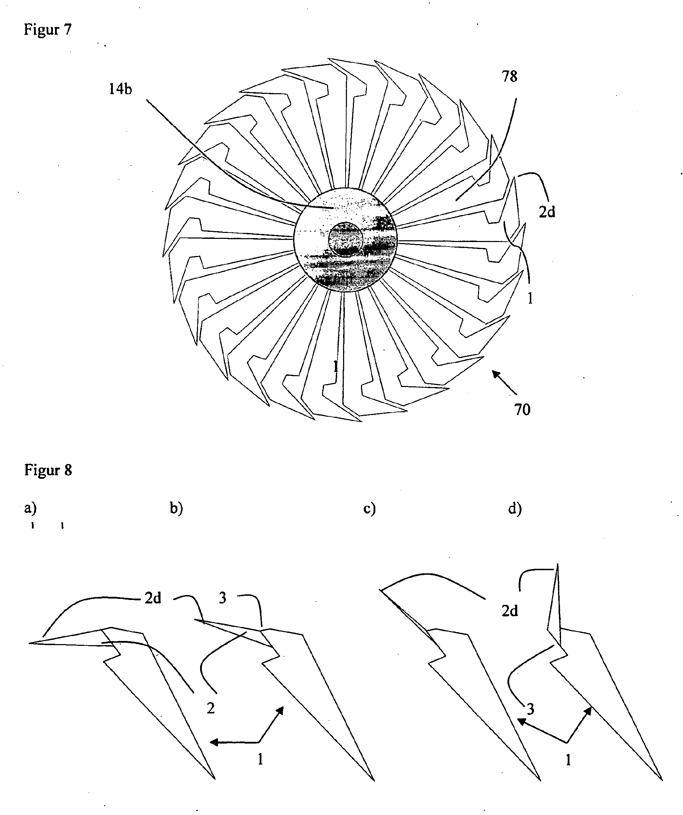

In Figur 7 ist eine weitere Möglichkeit gezeigt, wie mehrere Lanzetten (1) magaziniert werden können. Dies ist in Figur 7 in Form eines Multilanzettenrades (70) gezeigt, in denen die Lanzetten (1) in einer Ebene (89) angeordnet sind. In diesem Fall sind die Lanzettenspitzen (2 d) säbelförmig ausgearbeitet, so dass zum Benutzen der Lanzetten (1) ein seitliches Abknicken der Lanzettenspitze (2 d) notwendig ist. Hierzu ist bevorzugter Weise annähernd parallel zum Lanzettenkörper (78) eine Prägung im Bereich der Spitze (2) eingebracht (hier nicht gezeigt). Auf dem Lanzettenrad (70) kann zusätzlich ein Testelement angebracht sein (hier nicht gezeigt). Außerdem kann die Lanzette (1) als Microsampler ausgebildet sein. Hierzu weist die dann bevorzugt im Spitzenbereich (2) eine Struktur zur Aufnahme von Körperflüssigkeit auf. Ebenfalls nicht gezeigt ist eine weitere Prägung, die sich im Übergangsbereich vom Spitzenbereich (2) zum Lanzettenkörper (78) befinden kann, und ein weiteres Knicken der Lanzettenspitze (2) nach dem Stechvorgang ermöglicht. Hierdurch wird eine Übertragen der Körperflüssigkeit auf ein Testelement möglich, das sich ebenfalls auf dem Träger bzw. Lanzettenkörper befinden kann.

Das seitliche Abknicken ist in Figuren 8 A - D gezeigt. In Figur 8 A ist die Lanzette (1) im ungeknickten Zustand gezeigt. In den Figuren 8 B und C ist die Lanzette 1 beim Knickvorgang gezeigt, wobei die Lanzettenspitze (2d) sich langsam aus der Lanzettenkörperebene (89) herausbewegt und in einem bestimmten Winkel wie in Figur 8 D dargestellt ist, zum Einstechen bereit ist. Die Lanzette (1) wird bei dem Einstich kreisförmig um den Punkt (20) bewegt. Aufgrund dieser kreisförmigen Bewegung ist es vorteilhaft die Ausformung des abgeknickten Spitzenbereiches (2) auf die Kreisbewegung hin anzupassen, was bedeutet, dass die Kanten (10) und (11) so geformt sind, dass sie einen möglichst schmerzarmen Einstich bzw. Einschnitt in die Haut gewährleisten können. Aufgrund der Stellung des Spitzenbereiches (2) in Relation zum Bereich (8) der Lanzette (1) ist zu erkennen, dass bei Drehung der Lanzette (1) um den Punkt (20) herum die Kanten (10) und (11) senkrecht zur Schneidbewegung in die Haut eindringen, während bei der Benutzung einer Lanzette (1) wie in Figur 1 D beschrieben, die Kanten (10) und (11) mit einem Winkel der von 0 Grad abweicht, quer zur Einstichbewegung in die Haut eintrifft.

### Liste der Bezugszeichen

- 1:: Lanzette
- 1a:: geknickte Lanzette
- 2:: Spitzenbereich
- 2a:: 1. Seitenfläche
- 2b: 2. Seitenfläche
- 2c:: Fläche
- 2d: Lanzettenspitze
- 3, 3 A, 3 B, 3 C:: erste Prägung
- 4:: 2. Prägung
- 5:: 3. Prägung
- 6:: Mittellinie
- 7:: Proximales Ende
- 8:: Bereich, Fläche
- 9:: 2. Fläche
- 10:: 1. Seitenkante
- 11:: 2. Seitenkante
- 12:: Knickbereich
- 14:: Trägerband
- 14 a:: Trägerbandebene
- 14b:: Träger

- 15:: Führung
- 16:: Kante
- 18:: 1. Seitenkante
- 19:: 2. Seitenkante
- 20:: Lanzettenende
- 21:: Rolle
- 22:: Testfeld
- 30 A:: 1. Stössel
- 30 B:: 2. Stössel
- 39:: Spule
- 40:: Gerät
- 41:: Öffnung
- 42:: Detektor
- 43:: Knickelement
- 70:: Multilanzettenrad
- 78:: Lanzettenkörper
- 89:: Lanzettenebene

## Patentansprüche

1. Eine Vorrichtung zur Gewinnung von Körperflüssigkeit, beinhaltend:
ein im Wesentlichen planares Trägerband,
mindestens eine Lanzette, beinhaltend einen Lanzettenkörper und eine Spitze, wobei die Lanzette liegend auf dem Trägerband angeordnet ist, **dadurch gekennzeichnet, dass** die Lanzette einen Knickbereich beinhaltet, sodass die Lanzette unter Krafteinwirkung in dem Bereich der Spitze geknickt wird, sodass die Spitze in Bezug auf die Längsachse des Lanzettenkörpers in ihrer Ausrichtung verändert wird.

2. Vorrichtung nach Anspruch 1, **dadurch gekennzeichnet, dass** die mindestens eine Lanzette eine Flachlanzette ist.

3. Vorrichtung nach den Ansprüchen 1 oder 2, **dadurch gekennzeichnet, dass** beim Aufwenden einer Kraft auf den Lanzettenkörper, die Lanzettenspitze aus der im Wesentlichen planaren Ebene des Trägerbandes herausragt.

4. Vorrichtung nach einem der Ansprüche 1 bis 3, **dadurch gekennzeichnet, dass** die Lanzette im Knickbereich mindestens eine Struktur mit einer geänderten Steifigkeit besitzt.

5. Vorrichtung nach Anspruch 4, **dadurch gekennzeichnet, dass** bei Einwirkung einer Schwellenkraft auf die Lanzette, die mindestens eine Struktur mit veränderter Steifigkeit, ein Abknicken der angrenzenden Flächen bewirkt.

6. Vorrichtung nach einem der Ansprüche 1 bis 5, **dadurch gekennzeichnet, dass** die mindestens eine Lanzette in verschiedenen Ausrichtungen auf dem Trägerband angeordnet ist.

7. Vorrichtung nach einem der Ansprüche 1 bis 6, **dadurch gekennzeichnet, dass** auf dem Trägerband weiterhin mindestens ein Testelement angeordnet ist.

8. System zur Gewinnung von Körperflüssigkeit, mit einer Vorrichtung gemäß Anspruch 2 und einem Knickelement, das auf die Lanzette einwirkt, sodass die Lanzettenspitze unter Krafteinwirkung in Bezug auf die Längsachse der Lanzette in ihrer Ausrichtung veränderbar ist.

9. Vorrichtung nach Anspruch 8, **dadurch gekennzeichnet, dass** das Knickelement ein Regelelement beinhaltet, so dass die Lanzette an verschiedenen Stellen im Knickbereich geknickt werden kann.

10. Vorrichtung nach Anspruch 9, **dadurch gekennzeichnet, dass** die Lanzette auf einem Träger angeordnet ist, wobei bevorzugt der Träger im Wesentlichen scheibenförmig ausgestaltet ist.

11. Vorrichtung nach Anspruch 10, wobei die Lanzettenspitze die Form eines gekrümmten Säbels hat.

12. Vorrichtung nach Anspruch 10, **dadurch gekennzeichnet, dass** auf dem Träger weiterhin mindestens ein Testelement angeordnet ist, oder sich ein Testelement auf einem weiteren Träger befindet.

13. Vorrichtung nach Anspruch 12, **dadurch gekennzeichnet, dass** Lanzette und das Testelement so zueinander angeordnet sind, dass sie nach dem Knicken durch eine zweite Bewegung der Lanzette oder des Testelementes in Kontakt treten.

14. Vorrichtung nach einem der vorherigen Ansprüche, **dadurch gekennzeichnet, dass** die Lanzette, bevorzugterweise im Spitzenbereich beginnend, eine Struktur aufweist, die eine Aufnahme von Körperflüssigkeit ermöglicht.

## Claims

1. A device for obtaining body fluid, comprising:
an essentially planar carrier tape,
at least one lancet comprising a lancet body and a tip, where the lancet is arranged horizontally on the carrier tape, **characterized in that** the lancet comprises a bending region such that when a force acts on the lancet, it bends in the region of the tip such that the orientation of the tip is changed relative to the longitudinal axis of the lancet body.

2. Device according to claim 1, **characterized in that** the at least one lancet is a flat lancet.

3. Device according to claims 1 or 2, **characterized in that** the lancet tip projects out of the essentially planar plane of the carrier tape when a force is applied to the lancet body.

4. Device according to one of the claims 1 to 3, **characterized in that** the lancet has at least one structure having an altered stiffness in the bending region.

5. Device according to claim 4, **characterized in that** when a threshold force acts on the lancet, the at least one structure with an altered stiffness results in a bending of the adjoining areas.

6. Device according to one of the claims 1 to 5, **characterized in that** the at least one lancet is arranged on the carrier tape in various orientations.

7. Device according to one of the claims 1 to 6, **characterized in that** in addition at least one test element is arranged on the carrier tape.

8. System for obtaining body fluid with a device according to claim 2 and a bending element which acts on the lancet such that the orientation of the lancet tip can be changed relative to the longitudinal axis of the lancet when a force acts thereon.

9. Device according to claim 8, **characterized in that** the bending element comprises a control element such that the lancet can be bent at different places in the bending region.

10. Device according to claim 9, **characterized in that** the lancet is arranged on a carrier and the carrier has an essentially disk-shaped design.

11. Device according to claim 10, **characterized in that** the lancet tip has the shape of a curved sabre.

12. Device according to claim 10, **characterized in that** at least one test element is additionally arranged on the carrier, or a test element is located on an additional carrier.

13. Device according to claim 12, **characterized in that** the lancet and test element are arranged relative to one another such that they make contact after the bending as a result of a second movement of the lancet or of the test element.

14. Device according to one of the previous claims, **characterized in that** the lancet has a structure which preferably begins in the tip region and enables body fluid to be taken up.

## Revendications

1. Un dispositif d'obtention de liquide corporel, comprenant :
une bande de support essentiellement plane,
au moins une lancette comprenant un corps de lancette et une pointe, ladite lancette étant disposée à plat sur la bande de support, **caractérisé en ce que** la lancette comprend une zone de pliage de manière à plier la lancette dans la région de la pointe sous l'action d'une force de façon à modifier l'orientation de ladite pointe par rapport à l'axe longitudinal du corps de lancette.

2. Dispositif selon la revendication 1, **caractérisé en ce que** ladite au moins une lancette est une lancette plate.

3. Dispositif selon les revendications 1 ou 2, **caractérisé en ce que**, lorsqu'une force est exercée sur le corps de lancette, la pointe de la lancette fait saillie du plan essentiellement plan de la bande de support.

4. Dispositif selon une des revendications 1 à 3, **caractérisé en ce que** la lancette possède dans la zone de pliage au moins une structure avec une rigidité modifiée.

5. Dispositif selon la revendication 4, **caractérisé en ce que**, lorsqu'une force de seuil agit sur la lancette, ladite au moins une structure de rigidité modifiée provoque un pliage des surfaces avoisinantes vers le bas.

6. Dispositif selon une des revendications 1 à 5, **caractérisé en ce que** ladite au moins une lancette est disposée sur la bande de support selon différentes orientations.

7. Dispositif selon une des revendications 1 à 6, **caractérisé en ce qu'**en outre au moins un élément de test est disposé sur ladite bande de support.

8. Système d'obtention de liquide corporel, avec un dispositif selon la revendication 2 et un élément de pliage agissant sur la lancette de manière à pouvoir modifier, sous l'action d'une force, l'orientation de la pointe de la lancette sous l'action d'une force par rapport à l'axe longitudinal de la lancette.

9. Dispositif selon la revendication 8, **caractérisé en ce que** ledit élément de pliage comprend un élément de réglage, permettant de plier la lancette à différents endroits dans la zone de pliage.

10. Dispositif selon la revendication 9, **caractérisé en ce que** la lancette est disposée sur un support, ledit support étant conçu de préférence essentiellement sous forme de disque.

11. Dispositif selon la revendication 10, la pointe de la lancette ayant la forme d'un sabre courbe.

12. Dispositif selon la revendication 10, **caractérisé en ce qu'**en outre au moins un élément de test est disposé sur ledit support, ou un élément de test se trouve sur un autre support.

13. Dispositif selon la revendication 12, **caractérisé en ce que** la lancette et l'élément de test sont disposés l'un par rapport à l'autre de manière telle qu'ils entrent en contact après le pliage par un second mouvement de la lancette ou de l'élément de test.

14. Dispositif selon une des revendications précédentes, **caractérisé en ce que** la lancette, de préférence en débutant dans la région de la pointe, présente une structure permettant de recueillir du liquide corporel.
